# EUROPEAN PATENT APPLICATION

(11) **EP 2 716 628 A1**
(43) Date of publication of application: **09.04.2014**
(21) Application number: 12793417.2
(22) Date of filing: 30.05.2012
(51) Int. Cl.: C07C 271/28, C07C 269/04, C07C 269/06, C07C 281/02, C07D 261/04, C07B 61/00

(54) **METHOD OF MANUFACTURING ISOXAZOLINE COMPOUND**

(30) Priority: 03.06.2011 JP 2011125014; 31.01.2012 JP 2012017679
(71) Applicant: SUMITOMO CHEMICAL CO., LTD., Tokyo 104-8260 (JP)
(72) Inventor: KUMAMOTO, Koji, Misawa-shi Aomori 033-0022 (JP); UJIHARA, Kazuya, Takarazuka-shi Hyogo 665-8555 (JP)
(74) Representative: Vossius & Partner
(86) International application number: PCT/JP2012/064487
(87) International publication number: WO 2012/165650

(57) **Abstract**

A new method for manufacturing an isoxazoline compound represented by formula (9): 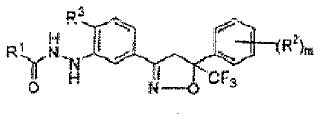 wherein R¹ represents a C1-C12 alkyl group optionally having one or more halogen atoms or a C3-C12 cycloalkyl group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another), and an intermediate for the manufacturing method are provided.

## Description

### TECHNICAL FIELD

The present invention relates to a method for producing an isoxazoline compound and a production intermediate thereof.

### BACKGROUND ART

It has been known that an isoxazoline compound represented by the following formula (9) is useful, for example, as an active ingredient of a pesticide. In addition, for example, WO2010/090344 discloses a method for synthesizing the isoxazoline compound represented by the following formula (9).

### DISCLOSURE OF THE INVENTION

The above conventional method is not necessarily satisfactory in the production of the isoxazoline compound represented by the following formula (9).

Therefore, an object of the present invention is to provide a new method that can produce the isoxazoline compound represented by the following formula (9) and a production intermediate thereof.

As a result of intensive studies the present inventors have found that the isoxazoline compound represented by the following formula (9) can be produced by reacting an aniline compound represented by the following formula (1) with di-tert-butyl dicarbonate to produce a carbamate compound represented by the following formula (2), reacting the obtained carbamate compound represented by the following formula (2) with monochloramine to produce a carbazate compound represented by the following formula (3), reacting the obtained carbazate compound represented by the following formula (3) with an acid chloride represented by the following formula (4) to produce an acylcarbazate compound represented by the following formula (5), reacting the obtained acyl carbazate compound represented by the following formula (5) with a tri fluoroacetophenone compound (6) represented by the following formula (6) to produce an aldol compound represented by the following formula (7), reacting the obtained aldol compound represented by the following formula (7) with di-tert-butyl dicarbonate, and then reacting the resultant with an acid to produce an enone compound represented by the following formula (8), and reacting the obtained enone compound represented by the following formula (8) with hydroxylamine, whereby the present invention has been completed.

More specifically, the present invention is as described below.
[1] A carbazate compound represented by formula (3): 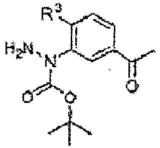
   [wherein R³ represents a halogen atom or a hydrogen atom].
[2] An acylcarbazate compound represented by formula (5): 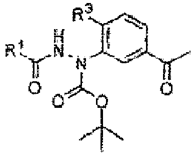
   [wherein R¹ represents a C1-C12 alkyl group optionally having one or more halogen atoms or a C3-C12 cycloalkyl group optionally having one or more halogen atoms, and R³ represents a halogen atom or a hydrogen atom].
[3] An aldol compound represented by formula (7): 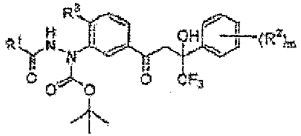
   [wherein R¹ represents a C1-C12 alkyl group optionally having one or more halogen atoms or a C3-C12 cycloalkyl group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)].
[4] An encore compound represented by formula (8): 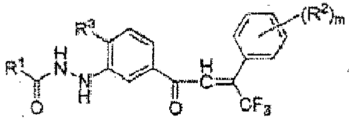
   [wherein R¹ represents a C1-C12 alkyl group optionally having one or more halogen atoms or a C3-C12 cycloalkyl group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)].
[5] tert-Butyl 5-acetyl-2-chlorophenylcarbamate.
[6] A method for producing a carbamate compound represented by formula (2): 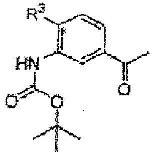
   [wherein R³ represents a halogen atom or a hydrogen atom] (hereinafter referred to as carbamate compound (2)), comprising a step of reacting an aniline compound represented by formula (1):

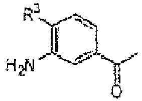

   [wherein R³ represents the same meaning as above] (hereinafter referred to as aniline compound (1)) with di-tert-butyl dicarbonate.
[7] A method for producing a carbazate compound represented by formula (3): 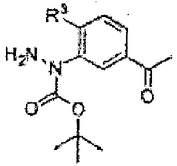
   [wherein R³ represents a halogen atom or a hydrogen atom] (hereinafter referred to as carbazate compound (3)), comprising a step of reacting the carbamate compound (2) with monochloramine.
[8] A method for producing an acylcarbazate compound represented by formula (5): 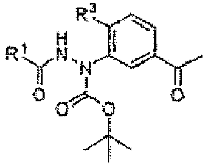
   [wherein R¹ represents a C1-C12 alkyl group optionally having one or more halogen atoms or a C3-C12 cycloalkyl group optionally having one or more halogen atoms, and R³ represents a halogen atom or a hydrogen atom] (hereinafter referred to as acylcarbazate compound (5)), comprising a step of reacting the carbazate compound (3) with an acid chloride represented by formula (4):

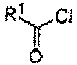

   [wherein R¹ represents a C1-C12 alkyl group optionally having one or more halogen atoms or a C3-C12 cycloalkyl group optionally having one or more halogen atoms] (hereinafter referred to as acid chloride (4)).
[9] A method for producing an aldol compound represented by formula (7): 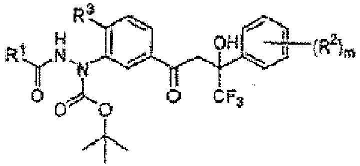
   [wherein R¹ represents a C1-C12 alkyl group optionally having one or more halogen atoms or a C3-C12 cycloalkyl group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)] (hereinafter referred to as aldol compound (7)), comprising a step of reacting the acylcarbazate compound (5) with a trifluoroacetophenone compound represented by formula (6):

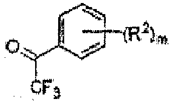

   [wherein R² represents a C1-C6 alkyl group optionally having one or more halogen atoms or a halogen atom, and m represents an integer of 0 to 5 (when m is an integer of 2 to 5, R²s may be different from one another)] (hereinafter referred to as trifluoroacetophenone compound (6)).
[10] A method for producing an enone compound represented by formula (8): 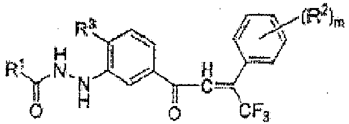
   [wherein R¹ represents a C1-C12 alkyl group optionally having one or more halogen atoms or a C3-C12 cycloalkyl group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)] (hereinafter referred to as enone compound (8)), comprising a step of reacting the aldol compound (7) with di-tert-butyl dicarbonate and then reacting the resultant with an acid.
[11] A method for producing an isoxazoline compound represented by formula (9): 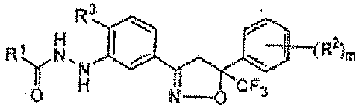
   [wherein R¹ represents a C1-C12 alkyl group optionally having one or more halogen atoms or a C3-C12 cycloalkyl group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)] (hereinafter referred to as isoxazoline compound (9)), comprising a step of reacting the enone compound (8) with hydroxylamine.
[12] A method for producing the isoxazoline compound (9) comprising steps of
   reacting the aniline compound (1) with di-tert-butyl dicarbonate to produce the carbamate compound (2), reacting the obtained carbamate compound (2) with monochloramine to produce the carbazate compound (3), reacting the obtained carbazate compound (3) with the acid chloride (4) to produce the acylcarbazate compound (5), reacting the obtained acylcarbazate compound (5) with the trifluoroacetophenone compound (6) to produce the aldol compound (7), reacting the obtained aldol compound (7) with di-tert-butyl dicarbonate and then reacting the resultant with an acid to produce the enone compound (8), and
   reacting the obtained enone compound (8) with hydroxylamine.

### EFFECT OF THE INVENTION

According to the present invention, a new method for producing the isoxazoline compound (9), a production intermediate thereof and the like can be provided.

### MODE FOR CARRYING OUT THE INVENTION

Examples of the "C1-C12 alkyl group optionally having one or more halogen atoms" represented by R¹ include a methyl group, an ethyl group, a propyl group, an isopropyl group, a butyl group, a tert-butyl group, an isobutyl group, a butyl group, a pentyl group, a hexyl group, a dodecyl group, a trifluoromethyl group, and a 3,3,3-trifluoropropyl group.

Examples of the "C3-C12 cycloalkyl group optionally having one or more halogen atoms" represented by R¹ include a cyclopropyl group, a cyclobutyl group, a cyclopentyl group, a cyclohexyl group, and a cycloheptyl group.

Examples of the "halogen atom" represented by R² include a fluorine atom, a chlorine atom, a bromine atom, and an iodine atom.

Examples of the "C1-C6 alkyl group optionally having one or more halogen atoms" represented by R² include a methyl group and a trifluoromethyl group.

Examples of the "halogen atom" represented by R³ include a fluorine atom, a chlorine atom, a bromine atom, and an iodine atom.

In the production of the carbamate compound (2) from the aniline compound (1) and di-tert-butyl dicarbonate, the carbamate compound (2) is produced by reacting the aniline compound (1) with di-tert-butyl dicarbonate.

The amount of the di-tert-butyl dicarbonate used for the reaction is usually 1 to 5 mol, based on 1 mol of the aniline compound (1).

The reaction can be carried out also in the presence of a base.

Examples of the base used for the reaction include organic bases such as pyridine, triethylamine, and M,N-dimethyl-4-aminopyridine. These bases can be used alone or in combination of two or more thereof.

The amount of the base used for the reaction is usually 0.001 to 10 mol, based on 1 mol of the aniline compound (1).

The reaction can be carried out also in a solvent.

Examples of the solvent used for the reaction include hydrocarbons such as toluene, ethers such as tetrahydrofuran, and halogenated hydrocarbons such as chloroform. These solvents can be used alone or in combination of two or more thereof.

The reaction temperature is usually in the range of -20 to 200°C or not more than the boiling point of the solvent used in the reaction.

The reaction time is usually in the range of 0.1 to 100 hours.

As mentioned above, the carbamate compound (2) can be produced. Further, it is also possible to purify the obtained carbamate compound by means such as extraction by separation, filtration, recrystallization, or column chromatography, as necessary.

In the production of the carbazate compound (3) from the carbamate compound (2) and monochloramine, the carbazate compound (3) is produced by reacting the carbamate compound (2) with monochloramine.

The reaction is usually carried out in the presence of a solvent.

Examples of the solvent used for the reaction include hydrocarbons such as toluene, ethers such as tetrahydrofuran and methyl tert-butyl ether, halogenated hydrocarbons such as chloroform, and water. These solvents can be used alone or in combination of two or more thereof.

The reaction can be carried out also in the presence of a phase transfer catalyst. Examples of the phase transfer catalyst used for the reaction include tetrabutylammonium bromide and trioctylmethylammonium chloride. These phase transfer catalysts can be used alone or in combination of two or more thereof.

The amount of the phase transfer catalyst used for the reaction is usually 0.001 to 1 mol, based on 1 mol of the carbamate compound (2).

The amount of the monochloramine used for the reaction is usually 1 to 10 mol, based on 1 mol of the carbamate compound (2).

The monochloramine used for the reaction can be usually obtained by the reaction of sodium hypochlorite with ammonia, in the presence of a metal hydroxide.

Examples of the metal hydroxide used for the reaction include sodium hydroxide.

The reaction temperature is usually in the range of -20 to 100°C or not more than the boiling point of the solvent used for the reaction.

The reaction time is usually in the range of 0.1 to 100 hours.

As mentioned above, the carbazate compound (3) can be produced. Further, it is also possible to purify the obtained carbazate compound by means such as extraction by separation, filtration, recrystallization, or column chromatography, as necessary.

In the production of the acylcarbazate compound (5) from the carbazate compound (3) and the acid chloride (4), the acylcarbazate compound (5) is produced by reacting the carbazate compound (3) with the acid chloride (4).

The amount of the acid chloride (4) used for the reaction is usually 1 mol to 10 mol, based on 1 mol of the carbazate compound (3).

The reaction is usually carried out in the presence of a base.

Examples of the base used for the reaction include organic bases such as pyridine, triethylamine, and N,N-dimethyl-4-aminopyridine. These bases can be used alone or in combination of two or more thereof.

The amount of the base used for the reaction is usually 0.1 to 10 mol, based on 1 mol of the carbazate compound (3).

The reaction is usually carried out in the presence of a solvent.

Examples of the solvent used for the reaction include hydrocarbons such as toluene, ethers such as tetrahydrofuran, and halogenated hydrocarbons such as chloroform. These solvents can be used alone or in combination of two or more thereof.

The reaction temperature is usually in the range of -20 to 100°C or not more than the boiling point of the solvent used for the reaction.

The reaction time is usually in the range of 0.1 to 100 hours.

As mentioned above, the acylcarbazate compound (5) can be produced. Further, it is also possible to purify the obtained acyl carbazate compound by means such as extraction by separation, filtration, recrystallization, or column chromatography, as necessary.

In the production of the aldol compound (7) from the acylcarbazate compound (5) and the trifluoroacetophenone compound (6), the aldol compound (7) is produced by reacting the acylcarbazate compound (5) with the trifluoroacetophenone compound (6).

The amount of the trifluoroacetophenone compound (6) used for the reaction is usually 0.1 to 10 mol, based on 1 mol of the acylcarbazate compound (5).

The reaction is usually carried out in the presence of a base.

Examples of the base used for the reaction include organic bases such as triethylamine and diazabicycloundecene (1,8-diazabicyclo[5.4.0]-7-undecene). These bases can be used alone or in combination of two or more thereof.

The amount of the base used for the reaction is usually 0-001 to 10 mol, based on 1 mol of the acylcarbazate compound (5).

The reaction is usually carried out in the presence of a solvent.

Examples of the solvent used for the reaction include ethers such as tetrahydrofuran. These solvents can be used alone or in combination of two or more thereof.

The reaction temperature is usually in the range of -20 to 200°C or not more than the boiling point of the solvent used for the reaction.

The reaction time is usually in the range of 0.1 to 100 hours.

As mentioned above, the aldol compound (7) can be produced. Further, it is also possible to purify the obtained aldol compound by means such as extraction by separation, filtration, recrystallization, or column chromatography, as necessary.

In the production of the enone compound (8) from the aldol compound (7), di-tert-butyl dicarbonate and an acid, the enone compound (8) is produced by reacting the aldol compound (7) with di-tert-butyl dicarbonate and then reacting the resultant with an acid.

The amount of di-tert-butyl dicarbonate used for the reaction is usually 1 to 10 mol, based on 1 mol of the aldol compound (7).

The reaction is usually divided into Step 1 for reacting the aldol compound (7) with di-tert-butyl dicarbonate, and Step 2 for reacting the resultant with an acid.

Step 1 is usually carried out in the presence of a base.

Examples of the base used for the step include organic bases such as triethylamine and N,N-dimethyl-4-aminopyridine. These bases can be used alone or in combination of two or more thereof.

The amount of the base used in the step is usually 0.1 to 10 mol, based on 1 mol of the aldol compound (7).

The step is usually carried out in the presence of a solvent.

Examples of the solvent used in the step include hydrocarbons such as toluene, ethers such as tetrahydrofuran, and halogenated hydrocarbons such as chloroform. These solvents can be used alone or in combination of two or more thereof.

The reaction temperature of the step is usually in the range of -20 to 200°C or not more than the boiling point of the solvent used in the reaction.

The reaction time of the step is usually in the range of 0.1 to 100 hours.

It is also possible to have a step of distilling off the solvent from the reaction mixture between Step 1 and Step 2.

Examples of the acid used in Step 2 include trifluoroacetic acid.

The amount of the acid used in the step is usually 1 mol to 100 mol, based on 1 mol of the aldol compound (7).

The reaction temperature of the step is usually in the range of -20 to 100°C or not more than the boiling point of the solvent or the acid used in the reaction.

The reaction time of the step is usually in the range of 0.1 to 100 hours.

As mentioned above, the enone compound (8) can be produced. Further, it is also possible to purify the obtained encore compound by means such as extraction by separation, filtration, recrystallization, or column chromatography, as necessary.

In the production of the isoxazoline compound (9) from the enone compound (8), the isoxazoline compound (9) is produced by reacting the enone compound (8) with hydroxylamine.

The amount of hydroxylamine used for the reaction is usually 1 mol to 10 mol, based on 1 mol of the enone compound (8).

The reaction is usually carried out in the presence of a base.

Examples of the base used for the reaction include metal hydroxides such as sodium hydroxide and organic bases such as diazabicycloundecene. These bases can be used alone or in combination of two or more thereof.

The amount of the base used for the reaction is usually 0.1 mol to 10 mol, based on 1 mol of the enone compound (8).

As the hydroxylamine used in the reaction, a hydroxylamine in the form of an aqueous solution or a salt such as hydroxylamine hydrochloride can be used. These hydroxylamines can be used alone or in combination of two or more thereof.

The reaction is usually carried out in the presence of a solvent. Examples of the solvent used in the reaction include hydrocarbons such as toluene, ethers such as methyl tert-butyl ether and water, and these solvents can be used alone or in combination of two or more thereof.

The reaction can be carried out also in the presence of a phase transfer catalyst. Examples of the phase transfer catalyst used in the reaction include tetrabutylammonium bromide. These phase transfer catalysts can be used alone or in combination of two or more thereof.

The reaction temperature is usually in the range of -20 to 200°C or not more than the boiling point of the solvent used in the reaction.

The reaction time is usually in the range of 0.1 to 100 hours.

As mentioned above, the isoxazoline compound (9) can be produced. Further, it is also possible to purify the obtained isoxazoline compound by means such as extraction by separation, filtration, recrystallization, or column chromatography, as necessary.

Some of the aniline compounds (1) are commercially available, and other compounds can be produced according to the method described in Annalen der Chemie, Justus Liebigs vol. 81, p. 641 (1961).

Some of the acid chlorides (4) are commercially avail abl e, and other compounds can be produced according to the method described in the Fourth Series of Experimental Chemistry, Vol. 22, p. 115 (Maruzen).

Some of the trifluoroacetophenone compounds (6) are commercially available, and other compounds can be produced according to the method described in Canadian Journal of Chemistry vol. 58, p. 2491 (1980) or Angewandte Chemie International Edition vol. 37, p. 820 (1998).

Specific examples of the carbazate compound (3) are shown below.

Carbazate compounds represented by formula (3): 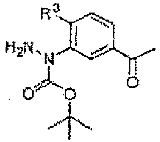
[wherein R³ represents the combination described in Table 1 bellow].

**[Table 1]**

| No. | R³ |
|---|---|
| 1 | H |
| 2 | F |
| 3 | Cl |
| 4 | Br |
| 5 | I |

Specific examples of the acylcarbazate compound (5) are shown below.

Acylcarbazate compounds represented by formula (5): 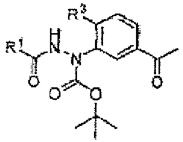
[wherein R¹ and R³ represent the combination described in Table 2 below].

**[Table 2]**

| No. | R¹ | R³ |
|---|---|---|
| 1 | CH₃ | Cl |
| 2 | CH₂CH₃ | Cl |
| 3 | CH₂CH₂CH₃ | Cl |
| 4 | CH(CH₃)₂ | Cl |
| 5 | C(CH₃)₃ | Cl |
| 6 | CH₂CH(CH₃)₂ | Cl |
| 7 | CF₃ | Cl |
| 8 | CH₂CH₂CF₃ | Cl |
| 9 | CH₂CH₂CF₃ | H |
| 10 | CH₂CH₂CF₃ | F |
| 11 | Cyclopropyl | H |
| 12 | Cyclopropyl | F |
| 13 | CH₃ | H |
| 14 | CH₃ | F |
| 15 | CH₃ | Br |
| 16 | Cyclopropyl | Cl |
| 17 | Cyclobutyl | Cl |
| 18 | Cyclopentyl | Cl |
| 19 | Cyclohexyl | Cl |
| 20 | Cycloheptyl | Cl |
| 21 | (CH₂)₃CH₃ | Cl |
| 22 | (CH₂)₄CH₃ | Cl |
| 23 | (CH₂)₅CH₃ | Cl |
| 24 | (CH₂)₁₁CH₃ | Cl |

Specific examples of the aldol compound (7) are shown below.

Aldol compounds represented by formula (7): 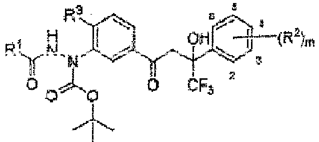
[wherein R¹, (R²)ₘ, and R³ represent the combination described in Table 3 below]

**[Table 3]**

| No. | R¹ | (R²)ₘ | R³ |
|---|---|---|---|
| 1 | CH₃ | 3-Cl, 5-Cl | Cl |
| 2 | CH₂CH₃ | 3-Cl, 5-Cl | Cl |
| 3 | CH₂CH₂CH₃ | 3-Cl, 5-Cl | Cl |
| 4 | CH(CH₃)₂ | 3-Cl, 5-Cl | Cl |
| 5 | C(CH₃)₃ | 3-Cl 5-Cl | Cl |
| 6 | CH₂CH(CH₃)₂ | 3-Cl, 5-Cl | Cl |
| 7 | CF₃ | 3-Cl, 5-Cl | Cl |
| 8 | CH₂CH₂CF₃ | 3-Cl, 5-Cl | Cl |
| 9 | CH₂CH₂CF₃ | 3-Cl, 5-Cl | H |
| 10 | CH₂CH₂CF₃ | 3-Cl, 5-Cl | F |
| 11 | Cyclopropyl | 3-Cl, 5-Cl | H |
| 12 | Cyclopropyl | 3-Cl, 5-Cl | F |
| 13 | CH₃ | 3-Cl, 5-Cl | H |
| 14 | CH₃ | 3-Cl, 5-Cl | F |
| 15 | CH₃ | 3-Cl, 5-Cl | Br |
| 16 | Cyclopropyl | 3-Cl, 5-Cl | Cl |
| 17 | Cyclobutyl | 3-Cl, 5-Cl | Cl |
| 13 | Cyclopentyl | 3-Cl, 5-Cl | Cl |
| 19 | Cyclohexyl | 3-Cl, 5-Cl | Cl |
| 20 | Cycloheptyl | 3-Cl, 5-Cl | Cl |
| 21 | (CH₂)₃CH₃ | 3-Cl, 5-Cl | Cl |
| 22 | (CH₂)₄CH₃ | 3-Cl, 5-Cl | Cl |
| 23 | (CH₂)₅CH₃ | 3-Cl, 5-Cl | Cl |
| 24 | (CH₂)₁₁CH₃ | 3-Cl, 5-Cl | Cl |
| 25 | CH₃ | 3-CF₃, 5-CF₃ | Cl |
| 26 | CH₃ | 3-Cl, 4-Cl, 5-Cl | Cl |

Specific examples of the enone compound (8) are shown below

Enone compounds represented by formula (8): 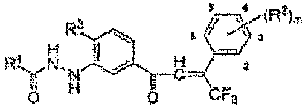
[wherein R¹, (R²)ₘ, and R³ represent the combination described in Table 4 below]

**[Table 4]**

| No. | R¹ | (R²)ₘ | R³ |
|---|---|---|---|
| 1 | CH₃ | 3-Cl, 5-Cl | Cl |
| 2 | CH₂Ch₃ | 3-Cl, 5-Cl | Cl |
| 3 | CH₂CH₂CH₃ | 3-Cl, 5-Cl | Cl |
| 4 | CH(CH₃)₂ | 3-Cl, 5-Cl | Cl |
| 5 | C(CH₃)₃ | 3-Cl, 5-Cl | Cl |
| 6 | CH₂CH(CH₃)₂ | 3-Cl, 5-Cl | Cl |
| 7 | CF₃ | 3-Cl, 5-Cl | Cl |
| 8 | CH₂CH₂CF₃ | 3-Cl 5-Cl | Cl |
| 9 | CH₂CH₂CF₃ | 3-Cl, 5-Cl | H |
| 10 | CH₂CH₂CF₃ | 3-Cl, 5-Cl | F |
| 11 | Cyclopropyl | 3-Cl, 5-Cl | H |
| 12 | Cyclopropyl | 3-Cl, 5-Cl | F |
| 13 | CH₃ | 3-Cl, 5-Cl | H |
| 14 | CH₃ | 3-Cl, 5-Cl | F |
| 15 | CH₃ | 3-Cl, 5-Cl | Br |
| 16 | Cyclopropyl | 3-Cl, 5-Cl | Cl |
| 17 | Cyclobutyl | 3-Cl, 5-Cl | Cl |
| 18 | Cyclopentyl | 3-Cl, 5-Cl | Cl |
| 19 | Cyclohexyl | 3-Cl, 5-Cl | Cl |
| 20 | Cycloheptyl | 3-Cl, 5-Cl | Cl |
| 21 | (CH₂)₃CH₃ | 3-Cl, 5-Cl | Cl |
| 22 | (CH₂)₄CH₃ | 3-Cl, 5-Cl | Cl |
| 23 | (CH₂)₅CH₃ | 3-Cl, 5-Cl | Cl |
| 24 | (CH₂)₁₁CH₃ | 3-Cl, 5-Cl | Cl |
| 25 | CH₃ | 3-CF₃, 5-CF₃ | Cl |
| 26 | CH₃ | 3-Cl, 4-Cl, 5-Cl | Cl |

Here, in (R²)ₘ of the above table, for example, the description of "3-Cl, 5-Cl" means that (R²)ₘ is substituents in the positions 3 and 5, m is 2, and R²s are each a chlorine atom.

### EXAMPLES

Examples of the present invention are shown below, but the present invention is not limited by these examples.

### Example 1

### Production of tert-butyl 5-acetyl-2-chlorophenylcarbamate

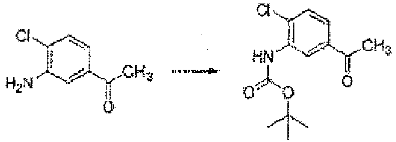

In tetrahydrofuran (100 ml) were dissolved 3-acetyl-6-chloroaniline (10.00 g) and di-tert-butyl dicarbonate (15.27 g), and N,N-dimethyl-4-aminopyridine (360 mg) was added thereto. After stirring at room temperature for 2 hours, di-tert-butyl dicarbonate (7.00 g) was added thereto, and the mixture was stirred for further 2 hours. The reaction mixture was concentrated under reduced pressure, and the resulting residue was dissolved in methanol (100 ml). Then, potassium carbonate (9.81 g) was added thereto, and the mixture was stirred at room temperature for 6 hours. This reaction mixture liquid was concentrated under reduced pressure, and the resulting residue was dissolved in methyl tert-butyl ether, and then the mixture was washed with water. The organic layer was dried over anhydrous sodium sulfate and then concentrated under reduced pressure. The resulting residue was subjected to silica gel column chromatography to obtain tert-butyl 5-acetyl-2-chlorophenylcarbamate (11.64 g).
¹H-NMR (CDCl₃) δ: 8.79 (1H, br s), 7.58 (1H, dd), 7.43 (1H, dd), 7.07 (1H, br s), 2.62 (3H, s), 1.56 (9H, s).

### Example 2

### Production of tert-butyl N-(5-acetyl-2-chlorophenyl) carbazate

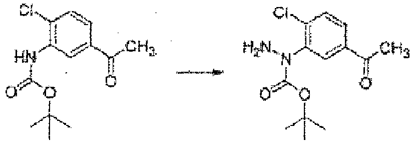

In tetrahydrofuran (39 ml) was dissolved tert-butyl 5-acetyl-2-chlorophenylcarbamate (3.72 g) produced in Example 1, and a 28% aqueous sodium hydroxide solution (40 ml), aqueous ammonia (13 ml), ammonium chloride (4.45 g) and trioctylmethylammonium chloride (758 mg) were added thereto. A 5% aqueous sodium hypochlorite solution (37 ml) was added dropwise to this mixture liquid at room temperature over 20 minutes, thereby producing monochloramine in the reaction system. After stirring at the same temperature for 12 hours, the organic layer was separated, then methyl tert-butyl ether was added to the aqueous layer, and the mixture was again extracted. The combined organic layers were dried over anhydrous sodium sulfate and then concentrated under reduced pressure. The resulting residue was subjected to silica gel column chromatography to obtain tert-butyl N-(5-acetyl-2-chlorophenyl)carbazate (2.60 g, Compound of Table 1-No. 3). ¹H-NMR (CDCl₃) δ: 7.90 (1H, br s), 7.81 (1H, dd), 7.51 (1H, d), 4.56 (2H, br s), 2.60 (3H, s), 1.40 (9H, br s).

### Example 3

### Production of tert-butyl N-(5-acetyl-2-chlorophenyl)-N'-(4,4,4-trifluorobutanoyl)carbazate

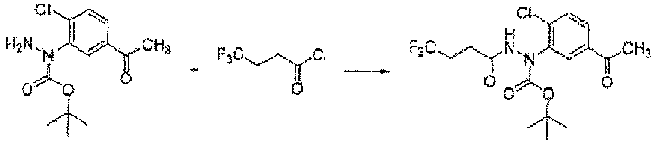

In tetrahydrofuran (15 ml) were dissolved tert-butyl N-(5-acetyl-2-chlorophenyl)carbazate (2.60 g) obtained in Example 2 and triethylamine (1097 mg), then 4,4,4-trifluorobutanoyl chloride (1741 mg) was added dropwise thereto under ice-cooling, and the mixture was stirred under ice-cooling for 30 minutes. An aqueous saturated sodium bicarbonate solution was added to the reaction mixture, and the mixture was extracted with ethyl acetate. The organic layer was dried over anhydrous sodium sulfate and then concentrated under reduced pressure. The resulting residue was subjected to silica gel column chromatography to obtain tert-butyl N-(5-acetyl-2-chlorophenyl)-N'-(4,4,4-trifluorobutanoyl) carbazate (3.30 g, Compound of Table 2-No. 8). ¹H-NMR (CDCl₃) δ: 8.21 (1H, br s), 7.88 (1H, dd), 7.78 (1H, br s), 7.52 (1H, d), 2.61-2.51 (7H, m), 1.52-1.42 (9H, m).

### Example 4

### Production of tert-butyl N-{2-chloro-5-[3-(3,5-dichlorophenyl)-4,4,4-trifluoro-3-hydroxybutanoyl]phenyl}-N'-(4,4,4-trifluorobutanoyl)carbazate

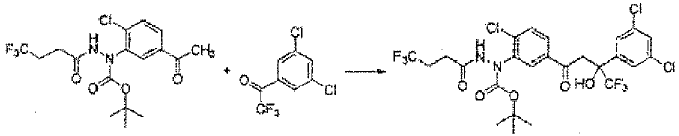

In tetrahydrofuran (5 ml) were dissolved tert-butyl N-(5-acetyl-2-chlorophenyl)-N'-(4,4,4-trifluorobutanoyl) carbazate (1.00 g) obtained in Example 3, triethylamine (496 mg) and α,α,α-trifluoro-3,5-dichloroacetophenone (892 mg), and the mixture was stirred at 60°C for 4 hours. This reaction mixture liquid cooled to room temperature was concentrated under reduced pressure, and the resulting residue was subjected to silica gel column chromatography to obtain tert-butyl N-{2-chloro-5-[3-(3,5-dichlorophenyl)-4,4,4-trifluoro-3-hydroxybutanoyl]phenyl}-N'-(4,4,4-trifluorobutanoyl) carbazate (1437 mg, Compound of Table 3-No. 8). ¹H-NMR (CDCl₃) δ: 8.25-8.22 (1H, br m), 7.91-7.84 (2H, m), 7.60-7.35 (4H, m), 5.54-5,49 (1H, m), 3.81-3.76 (1H, m), 3.66 (1H, d,), 2.75-2.52 (4H, m), 1.49-1.43 (9H, m).

### Example 5

### Production of N'-{2-chloro-5-[5-(3,5-dichlorophenyl)-5-trifluoromethyl-4,5-dihydroisoxazol-3-yl]phenyl}-(4,4,4-trifluorobutanoyl)hydrazide

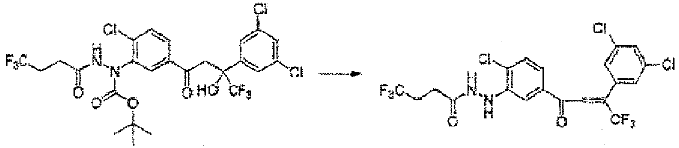

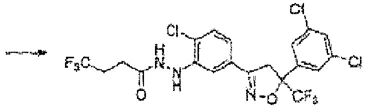

In tetrahydrofuran (2 ml) were dissolved tert-butyl N-{2-chloro-5-[3-(3,5-dichlorophenyl)-4,4,4-trifluoro-3-hydroxybutanoyl]phenyl}-N'-(4,4,4-trifluorobutanoyl) carbazate (326 mg) obtained in Example 4, triethylamine (51 mg) and N,N-dimethyl-4-aminopyridine (12 mg), and di-tert-butyl dicarbonate (131 mg) was added dropwise thereto at room temperature. After stirring at room temperature for 30 minutes, di-tert-butyl dicarbonate (185 mg) was added thereto, and the mixture was stirred at room temperature for further 1 hour. The reaction mixture was concentrated under reduced pressure, and trifluoroacetic acid (2.0 ml) was added to the resulting residue, then the mixture was stirred at room temperature for 30 minutes. This reaction mixture was concentrated under reduced pressure, and an aqueous saturated sodium bicarbonate solution was added thereto, and then the mixture was extracted with ethyl acetate. The organic layer was dried over anhydrous sodium sulfate and then concentrated under reduced pressure to obtain crude N'-{2-chloro-5-[3-(3,5-dichlorophenyl)-4,4,4-trifluoro-2-butenoyl]phenyl}-(4,4,4-trifluorobutanoyl)hydrazide (Compound of Table 4-No. 8). Melting point 82°C

The resulting crude N'-{2-chloro-5-[3-(3,5-dichlorophenyl)-4,4,4-trifluoro-2-butenoyl]phenyl}-(4,4,4-trifluorobutanoyl)hydrazide and tetrabutylammonium bromide (55 mg) were dissolved in methyl tert-butyl ether (1 ml). Thereto was added dropwise a solution obtained by dissolving hydroxylamine hydrochloride (69 mg) and sodium hydroxide (85 mg) in water (0.4 ml) under ice-cooling, and the mixture was stirred at room temperature for 1 hour. To the reaction mixture was added 2 M hydrochloric acid, and the mixture was extracted with ethyl acetate. The organic layer was dried over anhydrous sodium sulfate and then concentrated under reduced pressure, and the resulting residue was subjected to silica gel column chromatography to obtain N'-{2-chloro-5-[5-(3,5-dichlorophenyl)-5-trifluoromethyl-4,5-dihydroisoxazol-3-yl] phenyl}-(4,4,4-trifluorobutanoyl)hydrazide (168 mg). ¹H-NMR (CDCl₃) δ: 7.52-6.98 (7H, m), 6.50-6.45 (1H, m), 4.04-4.00 (1H, m), 3.66-3.62 (1H, m), 2.73-2.46 (4H, m).

### Example 6

### Production of tert-butyl N-(5-acetyl-2-chlorophenyl)-N'-cyclopropanecarbonylcarbazate

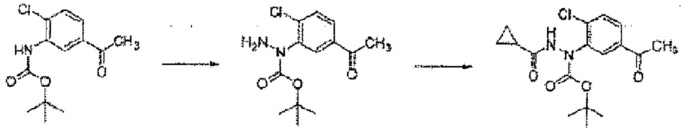

In 158 mL of methyl tert-butyl ether was dissolved 15.0 g of tert-butyl (5-acetyl-2-chlorophenyl)carbamate produced in Example 1, and 161 mL of a 28% aqueous sodium hydroxide solution, 52 mL of aqueous ammonia, 17.92 g of ammonium chloride and 3.06 g of trioctylmethylammonium chloride were added thereto. To this mixture liquid was added dropwise 149 mL of a 5% aqueous sodium hypochlorite solution at room temperature over 40 minutes. After stirring at the same temperature for 2 hours, the organic layer was separated, and the aqueous layer was extracted with methyl tert-butyl ether (80 mL × 2). The combined organic layers were washed sequentially with water (100 mL × 4) and a 10% aqueous sodium sulfite solution (100 mL × 3). The resulting organic layer was dried over anhydrous sodium sulfate and then concentrated under reduced pressure to obtain crude tert-butyl N-(5-acetyl-2-chlorophenyl)carbazate (Compound of Table 1-No. 3).

The resulting crude tert-butyl N-(5-acetyl-2-chlorophenyl)carbazate and 6.75 g of triethylamine were dissolved in 100 mL of tetrahydrofuran, and 6.96 g of cyclopropanecarbonyl chloride was added dropwise thereto under ice-cooling, and then the mixture was stirred under ice-cooling for 30 minutes. An aqueous saturated sodium bicarbonate solution was added to the reaction mixture, and the mixture was extracted with ethyl acetate. The organic layer was dried over anhydrous sodium sulfate and then concentrated under reduced pressure. The resulting residue was subjected to silica gel column chromatography to obtain tert-butyl N-(5-acetyl-2-chlorophenyl)-N'-cyclopropanecarbonylcarbazate (12.87 g, Compound of Table 2-No. 16).
¹H-NMR (CDCl₃) δ: 8.23 (1H, br s), 8.06 (1H, br s), 7.86 (1H, dd), 7.51 (1H, d), 2.58 (3H, s), 1.49-1.43 (10H, m), 1.05 (2H, br s), 0.86-0.84 (2H, br m).

### Example 7

### Production of tert-butyl N-{2-chloro-5-[3-(3,5-dichlorophenyl)-4,4,4-trifluoro-3-hydroxybutanoyl]phenyl}-N'-cyclopropanecarbonylcarbazate

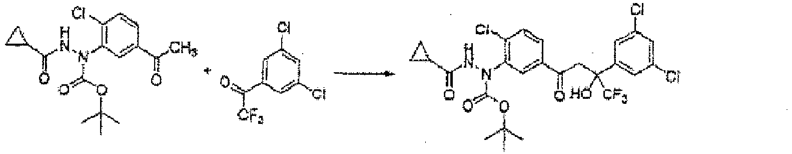

In 57 mL of tetrahydrofuran were dissolved 10.0 g of tert-butyl N-(5-acetyl-2-chlorophenyl)-N'-cyclopropanecarbonylcarbazate obtained in Example 6, 5.74 g of triethylamine and 10.33 g of 3',5'-dichloro-2,2,2-trifluoroacetophenone, and the mixture was stirred at 60°C for 8 hours. This reaction mixture liquid cooled to room temperature was concentrated under reduced pressure, and the resulting residue was subjected to silica gel column chromatography to obtain tert-butyl N-{2-chloro-5-[3-(3,5-dichlorophenyl)-4,4,4-trifluoro-3-hydroxybutanoyl] phenyl}-N'-cyclopropanecarbonylcarbazate (16.66 g, Compound of Table 3-No. 16).
¹H-NMR (CDCl₃) δ: 8.31-7.80 (3H, m), 7.62-7.35 (4H, m), 5.58 (1H, d), 3.79-3.68 (2H, m), 1.52-1.40 (10H, m), 1.07-1.06 (2H, br m), 0.89-0.87 (2H, br m).

### Example 8

### Production of N'-{2-chloro-5-[5-(3,5-dichlorophenyl)-5-trifluoromethyl-4,5-dihydroisoxazol-3-yl]phenyl} cyclopropanecarbohydrazide

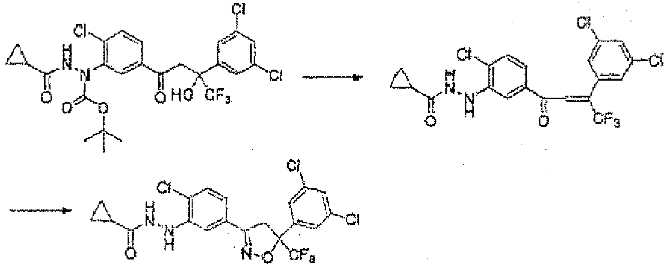

In 113 ml of tetrahydrofuran were dissolved 16.66 g of tert-butyl N-{2-chloro-5-[3-(3,5-dichlorophenyl)-4,4,4-trifluoro-3-hydroxybutanoyl)phenyl}-N'-cyclopropanecarbonyl carbazate obtained in Example 7, 2.86 g of triethylamine and 690 mg of N,N-diimethyl-4-aminopyridine, and 6.16 g of di-tert-butyl dicarbonate was added dropwise thereto at room temperature. After stirring at room temperature for 1 hour, 10.48 g of di-tert-butyl dicarbonate was further added dropwise thereto at room temperature, and the mixture was stirred at room temperature for 1 hour. The reaction mixture was concentrated under reduced pressure, and 50 ml of trifluoroacetic acid was added to the resulting residue, and then the mixture was stirred at room temperature for 30 minutes. This reaction mixture was concentrated under reduced pressure, and an aqueous saturated sodium bicarbonate solution was added thereto, and then the mixture was extracted with ethyl acetate. The organic layer was dried over anhydrous sodium sulfate and then concentrated under reduced pressure to obtain crude N'-{2-chloro-5-[3-(3, 5-dichlorophenyl)-4,4,4-trifluoro-2-butenoyl]phenyl} cyclopropanecarbohydrazide (Compound of Table 4-No. 16).
¹H-NMR (400 MHz, CDCl₃) δ 0.92 (2H, m), 1.06 (2H, m), 1.24 (1H, m), 3.23 (1H, s), 6.53 (1H, s), 7.15-7.48 (7H, m).

The resulting crude N'-{2-chloro-5-[3-(3,5-dichlorophenyl)-4,4,4-trifluoro-2-butenoyl]phenyl} cyclopropanecarbohydrazide and 3.10 g of tetrabutylammonium bromide were dissolved in 50 ml of methyl tert-butyl ether and 20 mL of tetrahydrofuran. Thereto was added dropwise a solution obtained by dissolving 3.93 g of hydroxylamine hydrochloride and 4.79 g of sodium hydroxide in 22.6 mL of water under ice-cooling, and the mixture was stirred at room temperature for 1 hour. To the reaction mixture was added 2 N hydrochloric acid to neutralize the reaction mixture, and the mixture was extracted with ethyl acetate. The organic layer was dried over anhydrous sodium sulfate and then concentrated under reduced pressure, and the resulting residue was subj ected to silica gel column chromatography to obtain 8.42 g of N'-{2-chloro-5-[5-(3,5-dichlorophenyl)-5-trifluoromethyl-4,5-dihydroisoxazol-3-yl]phenyl}cyclopropanecarbohydrazide.
¹H-NMR (400 MHz, CDCl₃) δ 0.92 (2H, m), 1.07 (2H, m), 1.53 (1H, m), 3.65 (1H, d), 4.04 (1H, d), 6.53 (1H, br s), 6.94-7.49 (7H, m).

## Claims

1. A carbazate compound represented by formula (3): 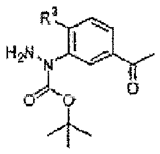
[wherein R³ represents a halogen atom or a hydrogen atom].

2. An acylcarbazate compound represented by formula (5): 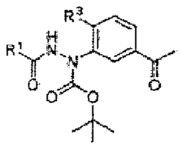
[wherein R¹ represents a C1-C12 alkyl group optionally having one or more halogen atoms or a C3-C12 cycloalkyl group optionally having one or more halogen atoms, and R³ represents a halogen atom or a hydrogen atom].

3. An aldol compound represented by formula (7): 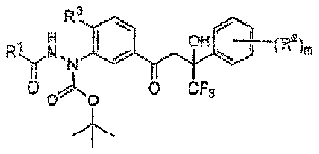
[wherein R¹ represents a C1-C12 alkyl group optionally having one or more halogen atoms or a C3-C12 cycloalkyl group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)].

4. An enone compound represented by formula (8): 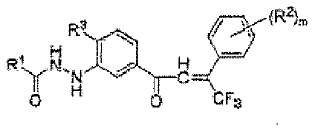
[wherein R¹ represents a C1-C12 alkyl group optionally having one or more halogen atoms or a C3-C12 cycloalkyl group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)].

5. tert-Butyl 5-acetyl-2-chlorophenylcarbamate.

6. A method for producing a carbamate compound represented by formula (2): 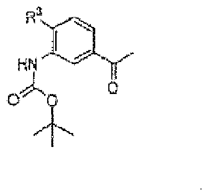
[wherein R³ represents a halogen atom or a hydrogen atom], comprising a step of reacting an aniline compound represented by formula (1): 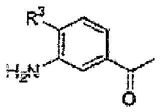
[wherein R³ is defined above]
with di-tert-butyl dicarbonate.

7. A method for producing a carbazate compound represented by formula (3): 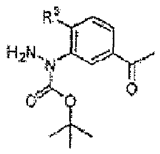
[wherein R³ represents a halogen atom or a hydrogen atom], comprising a step of reacting a carbamate compound represented by formula (2): 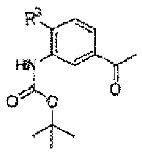
[wherein R³ is defined above]
with monochloramine.

8. A method for producing an acylcarbazate compound represented by formula (5): 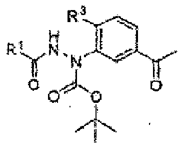
[wherein R¹ represents a C1-C12 alkyl group optionally having one or more halogen atoms or a C3-C12 cycloalkyl group optionally having one or more halogen atoms, and R³ represents a halogen atom or a hydrogen atom],
comprising a step of reacting a carbazate compound represented by formula (3): 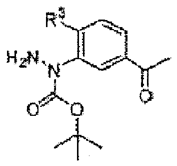
[wherein R³ is defined above]
with an acid chloride represented by formula (4): 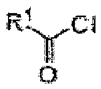
[wherein R¹ is defined above].

9. A method for producing an aldol compound represented by formula (7): 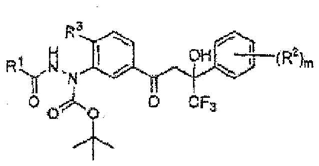
[wherein R¹ represents a C1-C12 alkyl group optionally having one or more halogen atoms or a C3-C12 cycloalkyl group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)], comprising a step of reacting an acylcarbazate compound represented by formula (5): 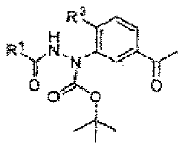
[wherein R¹ and R³ are defined above]
with a trifluoroacetophenone compound represented by formula (6): 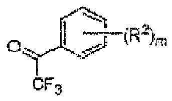
[wherein R² and m are defined above].

10. A method for producing an enone compound represented by formula (8): 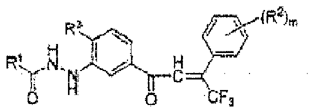
[wherein R¹ represents a C1-C12 alkyl group optionally having one or more halogen atoms or a C3-C12 cycloalkyl group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)], comprising a steps of reacting an aldol compound represented by formula (7): 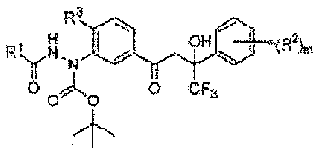
[wherein R¹, R², R³ and m are defined above] with di-tert-butyl di carbonate, and then reacting the resultant with an acid.

11. A method for producing an isoxazoline compound represented by formula (9): 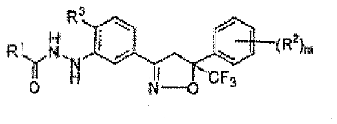
[wherein R¹ represents a C1-C12 alkyl group optionally having one or more halogen atoms or a C3-C12 cycloalkyl group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)], comprising a step of reacting an enone compound represented by formula (8): 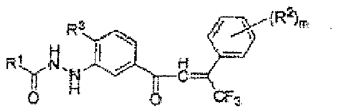
[wherein R¹, R², R³ and m are defined above]
with hydroxylamine.

12. A method for producing an isoxazoline compound represented by formula (9): 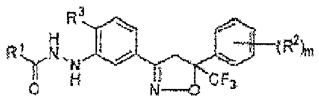
[wherein R¹ represents a C1-C12 alkyl group optionally having one or more halogen atoms or a C3-C12 cycloalkyl group optionally having one or more halogen atoms, R² represents a C1-C6 alkyl group optionally having one or more halogen atoms or a halogen atom, R³ represents a halogen atom or a hydrogen atom, and m represents an integer of 0 to 5 (when m is an integer of 2 to 5, R²s may be different from one another)], comprising steps of
reacting an aniline compound represented by formula (1); 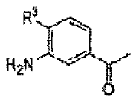
[wherein R³ is defined above]
with di-tert-butyl dicarbonate to produce a carbamate compound represented by formula (2): 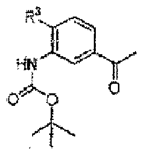
[wherein R³ is defined above],
reacting the obtained carbamate compound represented by the formula (2) with monochloramine to produce a carbazate compound represented by formula (3): 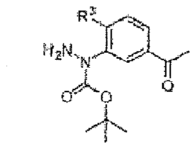
[wherein R³ is defined above],
reacting the obtained carbazate compound represented by the formula (3) with an acid chloride represented by formula (4): 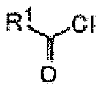
[wherein R¹ is defined above]
to produce an acylcarbazate compound represented by formula (5): 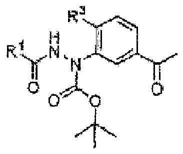
[wherein R¹ and R³ are defined above],
reacting the obtained acylcarbazate compound represented by the formula (5) with a trifluoroacetophenone compound represented by formula (6): 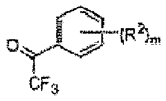
[wherein R² and m are defined above]
to produce an aldol compound represented by formula (7): 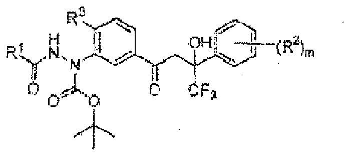
[wherein R¹, R², R³ and m are defined above],
reacting the obtained aldol compound represented by the formula (7) with di-tert-butyl dicarbonate and then reacting the resultant with an acid to produce an enone compound represented by formula (8): 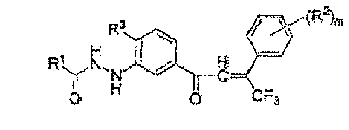
[wherein R¹, R², R³ and m are defined above],
and reacting the obtained enone compound represented by the formula (8) with hydroxylamine.
